(19) Europäisches Patentamt
European Patent Office
Office européen des brevets

(11) **EP 1 683 480 B1**

(12) **EUROPEAN PATENT SPECIFICATION**

(45) Date of publication and mention
of the grant of the patent:
**14.10.2009 Bulletin 2009/42**

(51) Int Cl.:
***A61B 5/053*** *(2006.01)* ***A61B 5/22*** *(2006.01)*

(21) Application number: **06000196.3**

(22) Date of filing: **05.01.2006**

(54) **Basal metabolic rate measuring device**

Grundumsatzmesseinrichtung

Dispositif de mesure du taux de métabolisme basal

(84) Designated Contracting States:
**DE FR GB**

(30) Priority: **21.01.2005 JP 2005014664**
**11.04.2005 JP 2005112973**

(43) Date of publication of application:
**26.07.2006 Bulletin 2006/30**

(73) Proprietor: **TANITA CORPORATION**
**Tokyo 174-0063 (JP)**

(72) Inventors:
• **Takehara, Tomoko**
**Itabashi-ku**
**Tokyo 174-0063 (JP)**
• **Honda, Yuka**
**Itabashi-ku**
**Tokyo 174-0063 (JP)**

(74) Representative: **Müller-Boré & Partner**
**Patentanwälte**
**Grafinger Strasse 2**
**81671 München (DE)**

(56) References cited:
**EP-A- 1 195 138** **EP-A- 1 279 366**
**EP-A- 1 279 367** **EP-A- 1 481 634**

• **NANCY F BUTTE, WILLIAM W WONG, MARGARITA S TREUTH, KENNETH J ELLIS AND E O?BRIAN SMITH: "Energy requirements during pregnancy based on total energy expenditure and energy deposition" AMERICAN JOURNAL OF CLINICAL NUTRITION, vol. 79, no. 6, June 2004 (2004-06), pages 1078-1087, XP002377805**
• **JM VAN RAAIJ, CM SCHONK, SH VERMAAT-MIEDEMA, ME PEEK AND JG HAUTVAST: "Body fat mass and basal metabolic rate in Dutch women before, during, and after pregnancy: a reappraisal of energy cost of pregnancy" AMERICAN JOURNAL OF CLINICAL NUTRITION, vol. 49, 1989, pages 765-772, XP002377806**

**Description**

BACKGROUND OF THE INVENTION

(i) Field of the Invention

The present invention relates to a basal metabolic rate measuring device capable of calculating a basal metabolic rate based on the fat free mass data and age data of a subject, particularly, to a basal metabolic rate measuring device capable of calculating the basal metabolic rate of a subject even when the subject is a pregnant woman.

(ii) Description of the Related Art

Accurate measurement of the basal metabolic rate of a subject requires a large-scale device such as a scentometer. Measurement of basal metabolic rate by use of a scentometer requires a subject to stay in a resting state for a long time with a mask or mouthpiece attached, causing a great burden on the subject. Further, the operation of the device and evaluations of measurement results require specialists.

Under the circumstances, the Ministry of Health, Labor and Welfare presents a basal metabolic rate obtained by multiplying a basal metabolic reference value based on gender and age by a body weight so that a statistically standard basal metabolic rate can be calculated, as presented in Nonpatent Literature 1, for example. It is used for simple estimation of basal metabolic rate.

Further, the present applicant has developed a method and device capable of calculating the basal metabolic rate of a subject easily based on the fat free mass and age of the subject. The method and device have already been practically used and also applied for patent, as exemplified by Patent Literatures 1 and 2.

The method of calculating and the device of measuring a basal metabolic rate that are disclosed in Patent Literatures 1 and 2 have been incorporated and practically used in, for example, a body fat meter and a body composition meter that use a so-called bioelectrical impedance method as disclosed in Patent Literatures 3 and 4 and calculate the basal metabolic rate of a subject by an estimation formula that takes fat free mass measured based on the bioelectrical impedance method as an important parameter.

Meanwhile, it is known that when a subject is a pregnant woman, the basal metabolic rate of the subject is increased by 20% to 30% in the latter half of the gestational period (pregnancy period) by the growth of a fetus and increases of organs associated with the fetus and the uterus. For example, Nonpatent Literature 2 discloses amounts of increase of oxygen consumption in organs in each week of the gestational period.

Further, the present applicant has confirmed that the body weight of fetus, the weight of amniotic fluid, the weight of placenta and the weight of the fetal part that is the total of these weights can be basically uniformly estimated based on the number of weeks of pregnancy. A body fat measuring device for pregnant women has been developed by use of the relationship between the number of weeks of pregnancy and the fetal part weight (intrauterine component weight) and the above bioelectrical impedance method and also applied for patent, as exemplified by Patent Literature 5.

Nonpatent Literature 1

"6th revised edition Nutritional Requirements of Japanese Reference Dietary Intake" edited by Health and Nutrition Information Association, published by Daiichi Shuppan Kabushiki Kaisha, September 10, 1999, pages 35 to 36

Nonpatent Literature 2

"Nutrition Guidance for Pregnant Women/Nutrition Guidance for Normal Pregnant Women 2 Energy" written by Masaharu Onaka, "MEDICUS LIBRARY 5 Nutrition Guidance Recommended for Mother and Child" edited by Motohiko Ichijo, published by MEDICA Shuppan, April 15, 1997, pages 40 to 44

Patent Literature 1

Japanese Patent Laid-Open Publication No. 2002-112982 Patent Literature 2
Japanese Patent Laid-Open Publication No. 2002-172099 Patent Literature 3
Japanese Patent Publication No. 1993-49050 Patent Literature 4
Japanese Patent No. 2,835,662 Patent Literature 5
Japanese Patent Laid-Open Publication No. 2003-102696

When a subject is a pregnant woman, her body is under a special condition. Therefore, it is not so desirable to use a scentometer which imposes a great burden on the subject for measurement of basal metabolic rate.

Further, although the calculation method as disclosed in Nonpatent Literature 1 which is presented by the Ministry of Health, Labor and Welfare and the calculation method and measurement device as disclosed in Patent Literatures 1 and 2 which have been developed by the present applicant are easy to use, they are intended for non-pregnant subjects. Thus, although results when they are used for pregnant women can be used as a useful reference, it is difficult to say that the results are accurate, and the methods and device are not suitable for measurement of the basal metabolic rate of a pregnant woman.

The present applicant has examined the accuracy of the calculation method presented by the Ministry of Health, Labor and Welfare and the calculation method and measurement device disclosed in Patent Literatures 1 and 2 with the help of pregnant women.

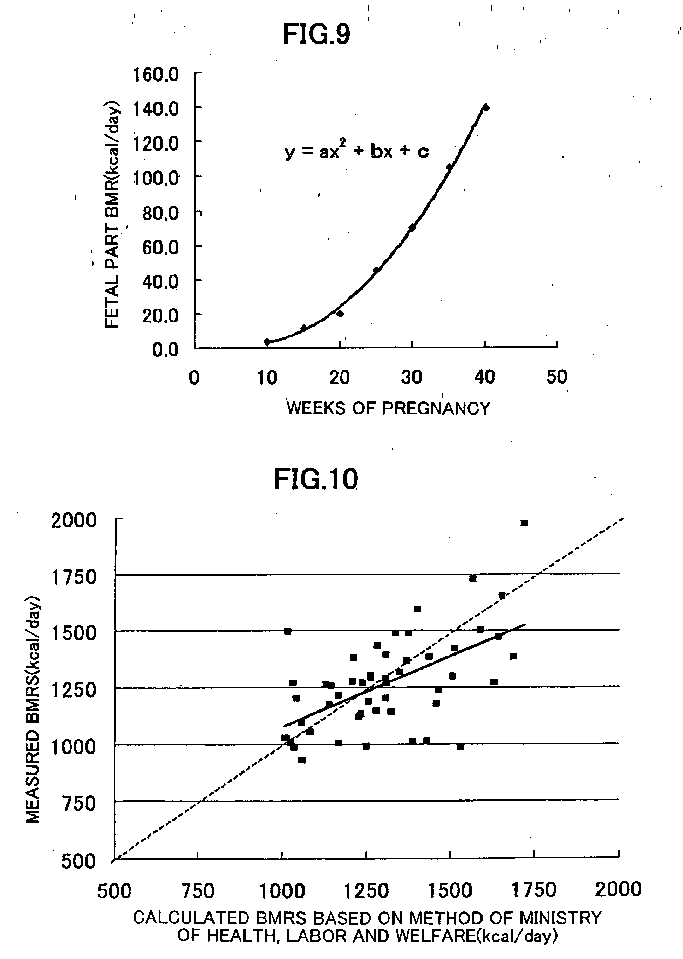

In Fig. 10, the horizontal axis represents basal metabolic rates calculated by use of the calculation method presented by the Ministry of Health, Labor and Welfare, the vertical axis represents basal metabolic rates measured by use of a scentometer, and the calculated basal metabolic rates and measured basal metabolic rates of the pregnant subjects are plotted as examination data. Broken lines in Fig. 10 indicate matching between values on the horizontal axis and values on the vertical axis. It is understood that it is difficult to calculate the basal metabolic rate of a pregnant woman accurately by this calculation method since the average line (solid line) obtained from the examination data of the subjects deviates from the broken lines.

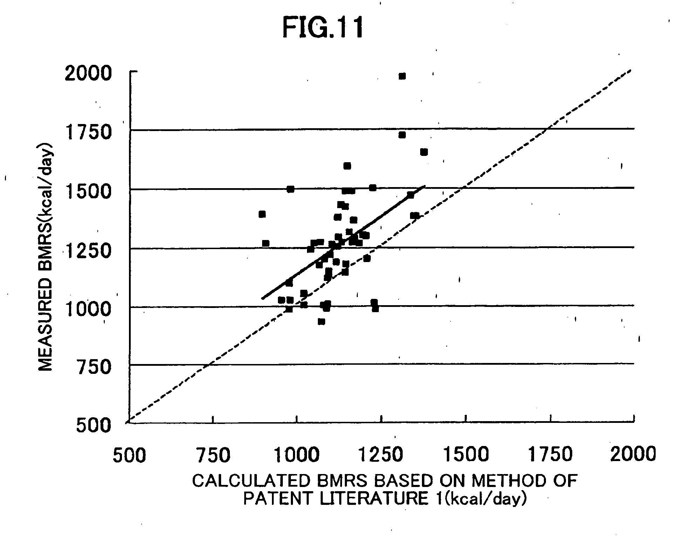

Further, in Fig. 11, the horizontal axis represents basal metabolic rates calculated by particular use of a calculation formula: BMR = A x FFM$^2$ + B x FFM + C x (1/AGE) + D x Wt + E (wherein BMR represents a basal metabolic rate, FFM represents fat free mass, AGE represents age, Wt represents a body weight, and A, B, C, D and E represent constants) out of calculation methods disclosed in Patent Literature 1, the vertical axis represents basal metabolic rates measured by use of a scentometer, and the calculated basal metabolic rates and measured basal metabolic rates of the pregnant subjects are plotted as examination data. As in the case of Fig. 10, broken lines in Fig. 11 indicate matching between values on the horizontal axis and values on the vertical axis. It is understood that it is also difficult to calculate the basal metabolic rate of a pregnant woman accurately even by this calculation formula since the average line (solid line) obtained from the examination data of the subjects deviates from the broken lines.

EP 1 195 138 A1 discloses an apparatus for measuring basal metabolism of a subject using the fat free mass, age and body weight of the subject.

EP 1 279 367 A1 discloses body fat measuring system and health care system for pregnant women.

It is the object of the invention to provide a basal metabolic rate measuring device having extended and improved measurement capabilities.

This object is fulfilled by a device having the features disclosed in claim 1 or claim 3. Preferred embodiments are defined in the dependent subclaims.

According to the present invention there is provided a basal metabolic rate measuring device capable of measuring the basal metabolic rate of a pregnant woman easily and accurately by improving a known basal metabolic rate measuring device capable of calculating a basal metabolic rate based on the fat free mass data and age data of a subject.

SUMMARY OF THE INVENTION

A basal metabolic rate measuring device of the present invention comprises:

> age data acquiring means,
> body height data acquiring means,
> body weight data acquiring means,
> impedance data acquiring means,
> fat free mass data acquiring means, and
> basal metabolic rate calculating means,
> and further comprising:

>> fetal part weight data acquiring means,
>> wherein
>> the age data acquiring means acquires the age data of a subject, the body height data acquiring means acquires the body height data of the subject,
>> the body weight data acquiring means acquires the body weight data of the subject,
>> the impedance data acquiring means acquires the bioelectrical impedance data of the subject,
>> the fetal part weight data acquiring means acquired the weight data of the fetal part of the subject when the subject is a pregnant woman,

the fat free mass data acquiring means calculates the fat free mass data of the body of the pregnant subject based on at least the acquired body height data, body weight data, impedance data and fetal part weight data, and the basal metabolic rate calculating means calculates the basal metabolic rate of the pregnant subject based on at least the fat free mass data and age data of mother.

In the basal metabolic rate measuring device of the present invention, the basal metabolic rate calculating means calculates the basal metabolic rate of the pregnant subject based on weight data obtained by adding the fetal part weight data to the fat free mass data of mother and the age data.

Further, the fetal part weight data acquiring means comprises gestational period data acquiring means for acquiring the data of time elapsed after pregnancy of the pregnant subject, and fetal part weight data determining means for determining the weight data of the fetal part based on the gestational period data.

Alternatively, the basal metabolic rate measuring device of the present invention further comprises:

gestational period data acquiring means, wherein the gestational period data acquiring means acquires the data of time elapsed after pregnancy of the pregnant subject, and the basal metabolic rate calculating means calculates the basal metabolic rate of the body of the pregnant subject based on the fat free mass data and age data of mother, calculates the basal metabolic rate of the fetal part based on the gestational period data and calculates the basal metabolic rate of the pregnant subject by totaling the basal metabolic rate of mother and the basal metabolic rate of the fetal part.

Further, the fetal part weight data acquiring means comprises fetal part weight data determining means for determining the weight data of the fetal part based on the gestational period data acquired by the gestational period data acquiring means.

In addition, the gestational period data acquiring means provided to the basal metabolic rate measuring device of the present invention comprises:

measurement date data acquiring means for acquiring measurement date data, expected date of confinement data acquiring means for acquiring the data of the expected date of confinement of the pregnant subj ect, and gestational period data determining means for determining the data of time elapsed after pregnancy based on these measurement date data and data of the expected date of confinement.

The basal metabolic rate measuring device of the present invention is a known basal metabolic rate measuring device comprising basal metabolic rate calculating means for calculating a basal metabolic rate based on the fat free mass data and age data of a subject with an additional feature that the device calculates the fat free mass data of the body of the pregnant subject and calculates the basal metabolic rate of the subject based on at least the fat free mass data and age data. The fat free mass data of mother is calculated by the known basal metabolic rate measuring device provided with fetal part weight data acquiring means based on at least the body height data, body weight data, impedance data and fetal part weight data of the subject. Thus, the basal metabolic rate measuring device of the present invention can measure the basal metabolic rate of a pregnant woman easily and accurately.

In the basal metabolic rate measuring device of the present invention, when the above basal metabolic rate calculating means calculates the basal metabolic rate of the pregnant subject based on weight data obtained by adding the fetal part weight data to the fat free mass data of mother and age data, the basal metabolic rate measuring device of the present invention can be achieved by use of an estimation formula obtained by merely making minor changes on an estimation formula used in the known basal metabolic rate measuring device.

Further, when the above fetal part weight data acquiring means comprises gestational period data acquiring means for acquiring the data of time elapsed after pregnancy of the pregnant subject and fetal part weight data determining means for determining the weight data of the fetal part based on the acquired gestational period data, the fetal part weight data used in calculations of the fat free mass and eventually acquired basal metabolic rate of mother can be determined based on the gestational period data such as the number of weeks of pregnancy, thereby making the basal metabolic rate measuring device of the present invention easier to use.

Alternatively, in the basal metabolic rate measuring device of the present invention, when the above basal metabolic rate calculating means calculates the basal metabolic rate of mother based on the fat free mass data and age data of mother, calculates the basal metabolic rate of the fetal part based on the gestational period data and calculates the basal metabolic rate of the pregnant subject by totaling the basal metabolic rate of mother and the basal metabolic rate of the fetal part, provided that the gestational period data acquiring means for acquiring the data of time elapsed after pregnancy of the pregnant subject is provided, the basal metabolic rate measuring device of the present invention can be achieved by a simple arithmetic processing program which comprises calculating the basal metabolic rate of mother by use of an estimation formula obtained by merely making minor changes on an estimation formula used in the known basal metabolic rate measuring device, calculating the basal metabolic rate of the fetal part based on the gestational period data such as the number of weeks of pregnancy and eventually totaling these basal metabolic rates.

Further, when the above fetal part weight data acquiring means comprises fetal part weight data determining means for determining the weight data of the fetal part based on the gestational period data acquired by the above gestational period data acquiring means, the fetal part weight data used in calculation of the fat free mass of mother can be determined based on the gestational period data such as the number of weeks of pregnancy, thereby making the basal metabolic rate measuring device of the present invention easier to use.

Further, when the gestational period data acquiring means provided to the basal metabolic rate measuring device of the present invention comprises measurement date data acquiring means for acquiring measurement date data, expected date of confinement data acquiring means for acquiring the data of the expected date of confinement of the pregnant subject and gestational period data determining means for determining the data of time elapsed after pregnancy based on the acquired measurement date data and data of the expected date of confinement, the gestational period data and the fetal part weight data based on the gestational period data are determined or the basal metabolic rate of the feta part is calculated from easily specifiable date data such as measurement date on which the basal metabolic rate measuring device of the present invention is used and expected date of confinement, thereby making the basal metabolic rate measuring device of the present invention easier to use.

BRIEF DESCRIPTION OF THE DRAWINGS

Fig. 1 is an external perspective view of a basal metabolic rate measuring device 1 as an example of the present invention.

Fig. 2 is an enlarged view of the operation section of the basal metabolic rate measuring device 1.

Fig. 3 is a schematic diagram illustrating the electric circuit configuration of the basal metabolic rate measuring device 1.

Fig. 4 is a flowchart of a control processing program to be executed in the basal metabolic rate measuring device 1 as a first example.

Fig. 5 is a diagram illustrating the relationship between the number of weeks of pregnancy and fetal part weight data that are used in the basal metabolic rate measuring device 1 as the first example.

Fig. 6 is a diagram illustrating the result of examining the accuracy of basal metabolic rate calculation formula for pregnant women that is used in the basal metabolic rate measuring device 1 as the first example.

Fig. 7 is a diagram illustrating an example of printout from the basal metabolic rate measuring device 1 as the first example.

Fig. 8 is a flowchart of a control processing program to be executed in a basal metabolic rate measuring device 1 as a second example.

Fig. 9 is a diagram illustrating the relationship between the number of weeks of pregnancy and the basal metabolic rate of fetal part which are used in the basal metabolic rate measuring device 1 as the second example.

Fig. 10 is a diagram illustrating the result of examining a basal metabolic rate calculation method presented by the Ministry of Health, Labor and Welfare.

Fig. 11 is a diagram illustrating the result of examining a basal metabolic rate calculation method presented by Patent Literature 1.

DETAILED DESCRIPTION OF THE PREFERRED EMBODIMENTS

A basal metabolic rate measuring device of the present invention comprises:

age data acquiring means,
body height data acquiring means,
body weight data acquiring means,
impedance data acquiring means,
fat free mass data acquiring means, and
basal metabolic rate calculating means,
and further comprising:

fetal part weight data acquiring means,

wherein

the age data acquiring means acquires the age data of a subject,

the body height data acquiring means acquires the body height data of the subject,

the body weight data acquiring means acquires the body weight data of the subject,

the impedance data acquiring means acquires the bioelectrical impedance data of the subject,

the fetal part weight data acquiring means acquired the weight data of the fetal part of the subject when the subject is a pregnant woman,

the fat free mass data acquiring means calculates the fat free mass data of the body of the pregnant subject based on at least the acquired body height data, body weight data, impedance data and fetal part weight data, and

the basal metabolic rate calculating means calculates the basal metabolic rate of the pregnant subject based on at least the fat free mass data and age data of mother.

In the basal metabolic rate measuring device of the present invention, the basal metabolic rate calculating means desirably calculates the basal metabolic rate of the pregnant subject based on weight data obtained by adding the fetal part weight data to the fat free mass data of mother and the age data.

Further, the fetal part weight data acquiring means desirably comprises gestational period data acquiring means for acquiring the data of time elapsed after pregnancy of the pregnant subject, and fetal part weight data determining means for determining the weight data of the fetal part based on the gestational period data.

Alternatively, the basal metabolic rate measuring device of the present invention desirably further comprises gestational period data acquiring means for acquiring the data of time elapsed after pregnancy of the pregnant subject, and the basal metabolic rate calculating means desirably calculates the basal metabolic rate of the body of the pregnant subject based on the fat free mass data and age data of mother, calculates the basal metabolic rate of the fetal part based on the gestational period data and calculates the basal metabolic rate of the pregnant subject by totaling the basal metabolic rate of mother and the basal metabolic rate of the fetal part.

Further, the fetal part weight data acquiring means desirably comprises fetal part weight data determining means for determining the weight data of the fetal part based on the gestational period data acquired by the gestational period data acquiring means.

In addition,

the gestational period data acquiring means provided to the basal metabolic rate measuring device of the present invention desirably comprises:

measurement date data acquiring means for acquiring measurement date data,

expected date of confinement data acquiring means for acquiring the data of the expected date of confinement of the pregnant subject, and

gestational period data determining means for determining the data of time elapsed after pregnancy based on these measurement date data and data of the expected date of confinement.

Example 1

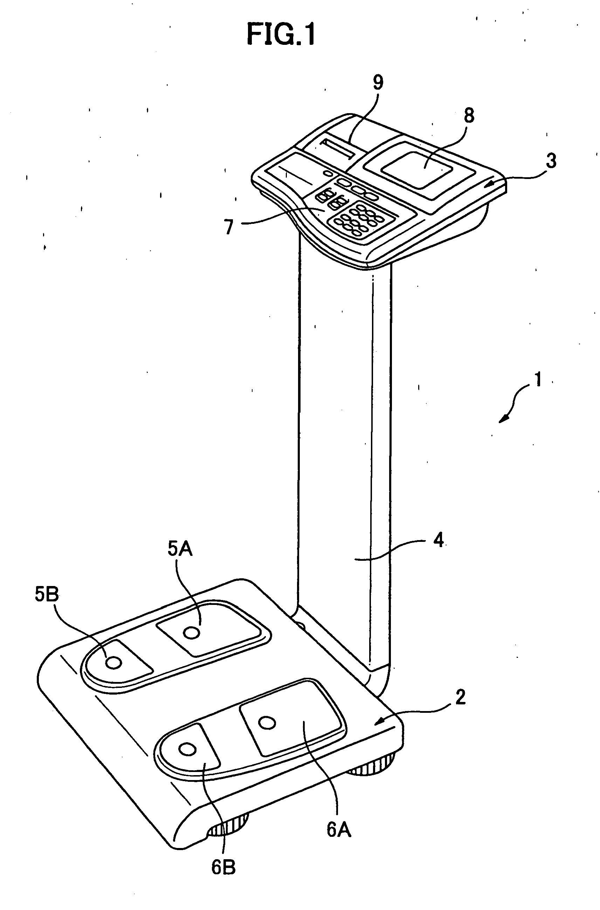

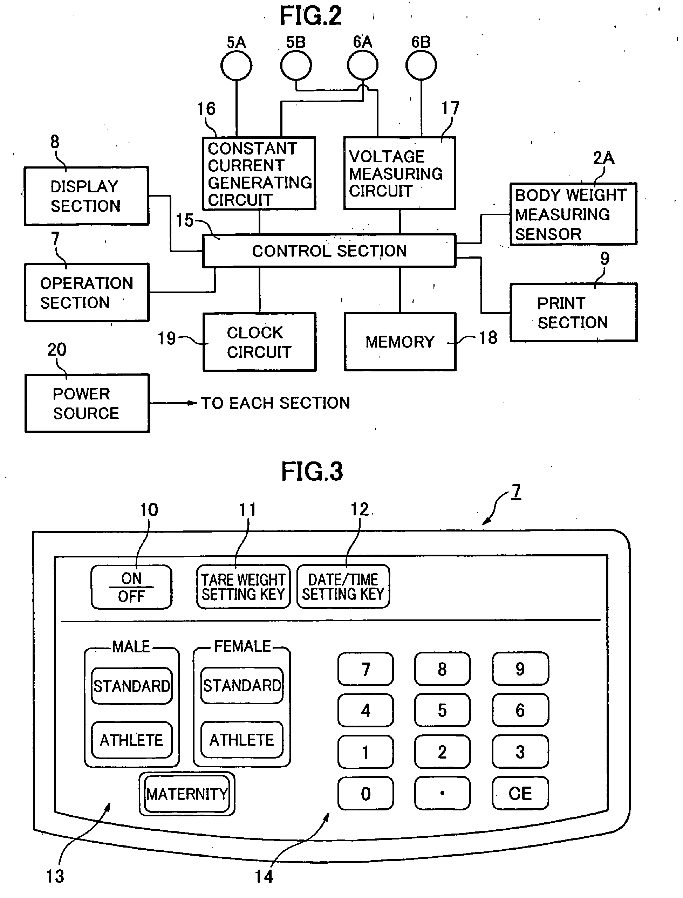

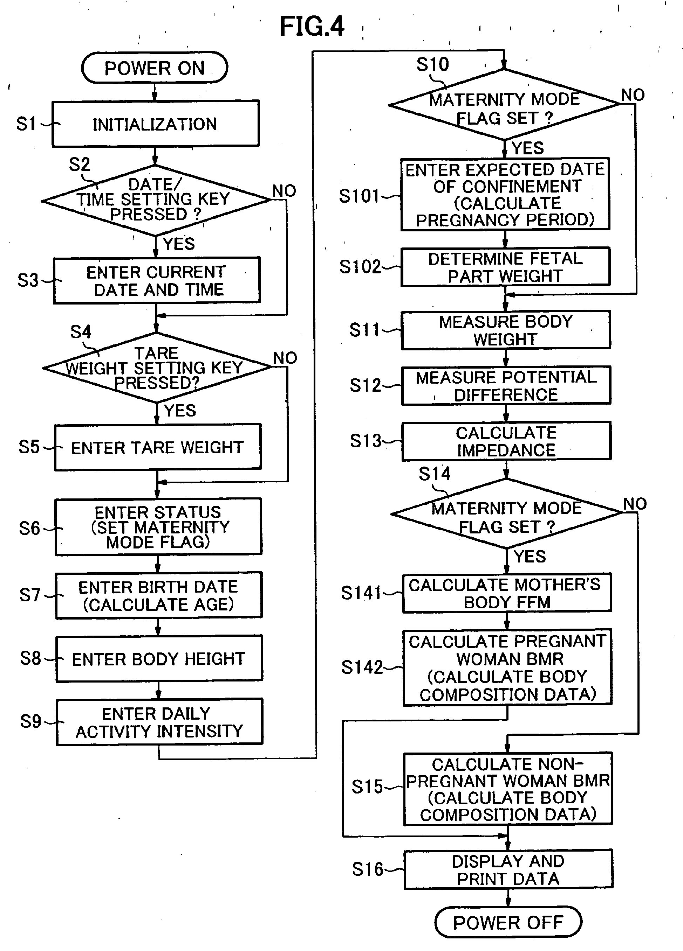

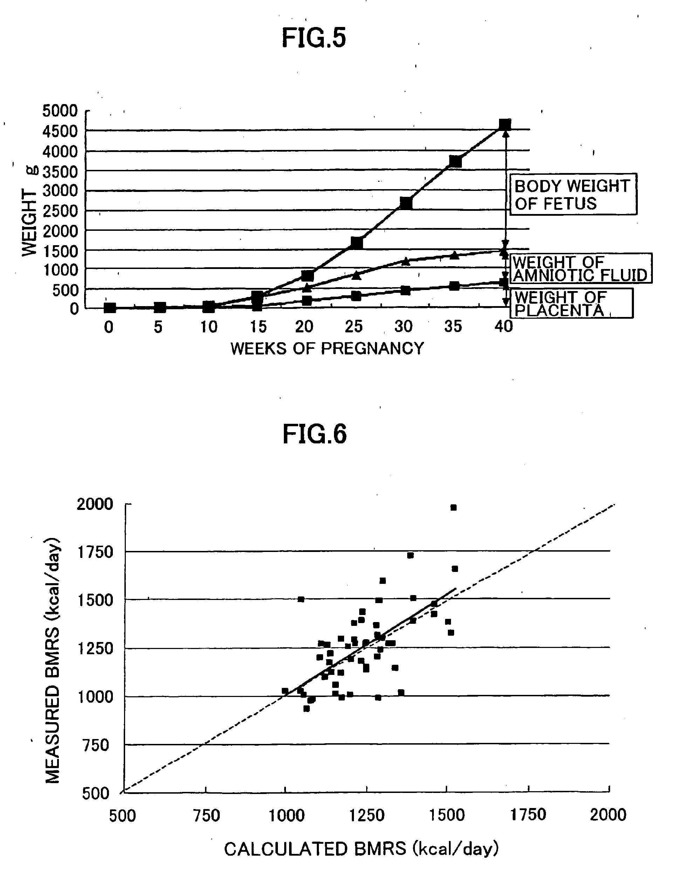

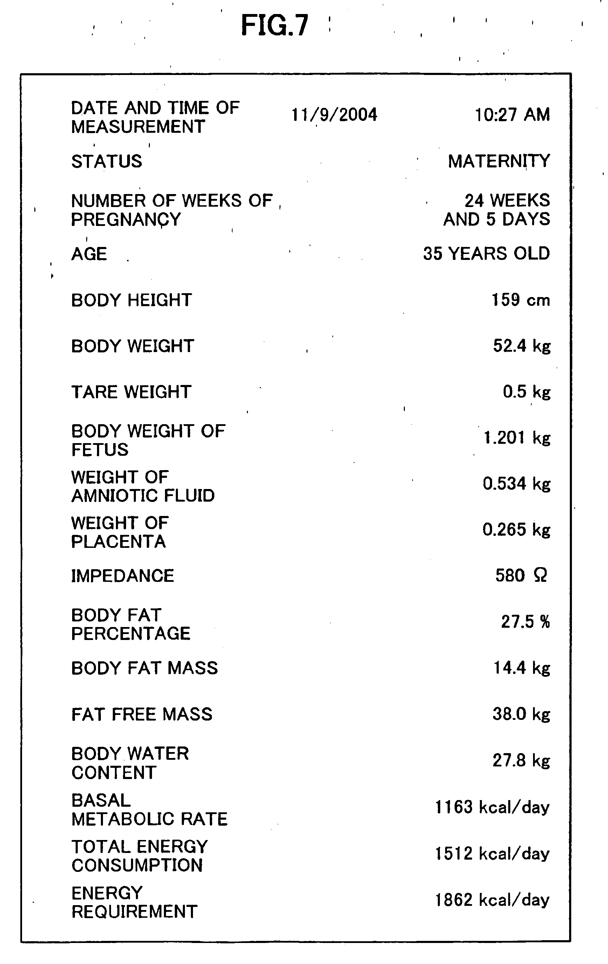

Hereinafter, a first example of the present invention will be described with reference to Figs. 1 to 7. Fig. 1 is an external perspective view of a basal metabolic rate measuring device 1 as an example of the present invention. Fig. 2 is an enlarged view of the operation section of the basal metabolic rate measuring device 1. Fig. 3 is a schematic diagram illustrating the electric circuit configuration of the basal metabolic rate measuring device 1. Fig. 4 is a flowchart of a control processing program to be executed in the basal metabolic rate measuring device 1 as the first example. Fig. 5 is a diagram illustrating the relationship between the number of weeks of pregnancy and fetal part weight data that are used in the basal metabolic rate measuring device 1 as the first example. Fig. 6 is a diagram illustrating the result of examining the accuracy of basal metabolic rate calculation formula for pregnant women that is used in the basal metabolic rate measuring device 1 as the first example. Fig. 7 is a diagram illustrating an example of printout from the basal metabolic rate measuring device 1 as the first example.

The basal metabolic rate measuring device 1 (hereinafter may be abbreviated simply as "device 1") that is an example of the present invention is a body composition meter capable of calculating various body composition data of a subject, such as body fat mass, fat free mass, a body water content, a muscle amount and bone mass, by use of a so-called bioelectrical impedance method. More specifically, the device 1 is an improved version of a body composition meter incorporating a known basal metabolic rate measuring device capable of calculating the basal metabolic rate of a subject based on the fat free mass data and age data of the subject.

The device 1 comprises a platform 2 on which a subject stands upright, an input/output unit 3 by which a subject or an operator (hereinafter may be generically referred to as "user") of the device inputs or outputs various data, and a support 4 which fixes the input/output unit 3 to the platform 2 and incorporates wires for electrically connecting them.

In the platform 2, a so-called load cell for detecting a load generated when a subject stands on the platform 2 is incorporated as a body weight measuring sensor 2A. Further, on the top surface of the platform 2, electrodes 5A and 5B that make contact with the bottom of the left foot of a subject and electrodes 6A and 6B that make contact with the bottom of the right foot of the subject are provided in an insulated state.

The input/output unit 3 incorporates an operation section 7 which comprises a switch panel for accepting operations of a user, a display section 8 which comprises a liquid crystal screen for displaying various data acquired by the device 1, and a print section 9 which comprises a thermal printer for printing out the data. On the switch panel of the operation section 7 are provided a power key 10 for turning on or off the device 1, a tare weight setting key 11 for setting the weight of the clothes of a subject, a date/time setting key 12 for setting a date and time, status setting keys 13 for setting the gender and body type of a subject or whether the subject is a pregnant woman, and a numeric keypad 14 for setting the body height, birth date and expected date of confinement of a subject.

Further, the input/output unit 3 also incorporates a control section 15 that comprises a processor and a temporary storage memory for executing various control processing programs to be described later, a constant current generating circuit 16 that is electrically connected to the above electrodes 5A and 6A to supply an alternating constant current to these electrodes, a voltage measuring circuit 17 that is electrically connected to the above electrodes 5B and 6B to measure a potential difference between these electrodes, a memory 18 that comprises a read-only mask memory storing the control processing programs to be executed by the control section 15 and a rewritable flash memory for storing the results of the control processings, and a clock circuit 19 for controlling the control processings in the control section 15. Further, the input/output unit 3 also incorporates a power source 20 for supplying electric power to be consumed by the control section 15 and other sections in the device 1.

The control section 15 is electrically connected to the body weight measuring sensor 2A, the operation section 7, the display section 8, the print section 9, the constant current generating circuit 16, the voltage measuring circuit 17, the memory 18 and the clock circuit 19. The control section 15 executes such control processings as measurement of the body weight of a subject, acceptance of input operations from the user, supply of current to the electrodes 5A and 6A, measurement of potential difference between the electrodes 5B and 6B, measurement of the bioelectrical impedance of the subject, calculations of body compositions based on the bioelectrical impedance, and display and printing of the results of the calculations.

Hereinafter, the control processings to be executed by the device 1 will be described with reference to the flowchart. When a user presses the power key 10 to activate the device 1, the control section 15 executes control processing described by the flowchart of Fig. 4.

In STEP S1, initialization processing is executed. More specifically, for example, temporary data of the last measurement which remains in the temporary storage memory of the control section 15 is deleted, and a maternity mode flag to be described later is cleared. At that time, in the control section 15, the current date and time data timed by the clock circuit 19 is acquired, the initial data (for example, 1.5 kg) of tare weight is retrieved and acquired from the memory 18, and they are displayed on the display section 8. Further, on the display section 8, a sign or message which urges the user to set the gender and body type of the subject is displayed.

In STEP S2, it is checked whether the date/time setting key 12 has been pressed. If it has been pressed, the control section 15 proceeds to STEP S3 and then to STEP S4, while if it has not been pressed, the control section 15 skips STEP S3 and proceeds to STEP S4. The user does not need to press the date/time setting key 12 unless there is a need to change the current date and time data displayed on the display section 8.

In STEP S3, the current date and time data is acquired again. That is, when the user enters date and time data by use of the numeric keypad 14, the control section 15 accepts the data entered from the numeric keypad 14, whereby the current date and time timed by the clock circuit 19 is replaced by the entered date and time data. Subsequently, the entered date and time data will be timed in the clock circuit 19 as the current date and time date.

In STEP S4, it is checked whether the tare weight setting key 11 has been pressed. If it has been pressed, the control section 15 proceeds to STEP S5 and then to STEP S6, while if it has not been pressed, the control section 15 skips STEP S5 and proceeds to STEP S6. The user does not need to press the tare weight setting key 11 unless there is a need to change the tare weight data displayed on the display section 8.

In STEP S5, the tare weight data of the subject is acquired again. That is, when the user enters tare weight data by use of the numeric keypad 14, the control section 15 accepts the data entered from the numeric keypad 14, whereby the initial data of tare weight read from the memory 18 is replaced by the entered tare weight data. The entered tare weight data is stored in the temporary storage memory as the actual tare weight of the subj ect and subj ected to measurement of body weight to be described later.

In STEP S6, the status data of the subject is acquired. That is, when the user selects and presses any one of 5 key switches constituting the status setting keys 13, i.e., "male standard", "male athlete", "female standard", "female athlete" and "maternity", the control section 15 accepts the input from the status setting keys 13, whereby the status data of the subject is determined according to the pressed key switch and stored in the temporary storage memory. In particular, when the "maternity" key switch has been pressed, a flag for executing the control processing of maternity mode to be described later is set. This flag has been cleared in advance in the above initialization processing of STEP S1.

In STEP S7, the age data of the subject is acquired. On the display section 8, a sign or message which urges the user to enter the birth date of the subject is displayed, and the control section 15 accepts input from the numeric keypad 14. When the user enters the birth date data of the subject by use of the numeric keypad 14, the age data of the subject is calculated based on the birth date data and the current date and time data and stored in the temporary storage memory. Alternatively, it is also possible to directly enter the age data in place of entering the birth date data.

Then, in STEP S8, the body height data of the subject is acquired. On the display section 8, a sign or message which urges the user to enter the body height of the subject is displayed, and the control section 15 accepts input from the numeric keypad 14. When the user enters the body height data of the subject by use of the numeric keypad 14, the body height data is stored in the temporary storage memory. In place of entering the body height data by the numeric keypad 14, it is also possible that an electronic body height meter comprising a position sensor using electricity or magnetism is attached to the device 1 and the control section 15 accepts body height data measured by the electronic body height meter.

Then, in STEP S9, the daily activity intensity data of the subject is acquired. On the display section 8, a sign or message which urges the user to enter the daily activity intensity data of the subject is displayed, and the control section 15 accepts input from the numeric keypad 14. When the user enters the daily activity intensity data of the subject by use of the numeric keypad 14, the daily activity intensity data is stored in the temporary storage memory. The daily activity intensity refers to indexed daily activities classified into different levels and is widely known as in the foregoing Nonpatent Literature 1.

Then, in STEP S10, it is checked whether the maternity mode flag is set. If it is already set, the control section 15 proceeds to STEPS S101 and S102 to be described later and then to STEP S11, while if it has been cleared, the control section 15 skips STEPS S101 and S102 and proceeds to STEP S11. That is, in this device 1, it is determined whether a subject is a pregnant woman based on the status of the flag set by a user by operating the status setting keys 13.

In STEP S11, the body weight data of the subject is acquired. That is, when the subject stands on the top surface of the platform 2 in the above status of STEP S10 or after completion of the process of STEP S102 to be described later, a load signal corresponding to the body weight of the subject is output from the body weight measuring sensor 2A. In fact, this STEP S11 is started by detecting the load signal in the control section 15. Then, a value resulting from subtracting the tare weight data from weight data corresponding to the detected load signal is stored in the temporary storage memory as the body weight data of the subject.

Then, in STEP S12, potential difference data occurring in the body of the subj ect due to the bioelectrical impedance of the subj ect is acquired. That is, when the subject stands on the top surface of the platform 2 in the above STEP S11, the bottom of the left foot makes contact with the electrodes 5A and 5B, and the bottom of the right foot makes contact with the electrodes 6A and 6B (or the subject stands so as to achieve the contact position). Then, an alternating constant current is supplied between both feet of the subject from the constant current generating circuit 16 through the electrodes 5A and 6A, and a potential difference corresponding to the impedance of the body occurs. The potential difference between both feet is detected by the voltage measuring circuit 17 through the electrodes 5B and 6B and stored in the temporary storage memory as potential difference data ascribable to a bioelectrical impedance. In this device 1, in addition to the above constitution in which the potential difference data between both feet is acquired by use of the electrodes 5A, 5B, 6A and 6B which make contact with the bottoms of the left and right feet of the subject, it is also possible that potential difference data between both hands is acquired by use of electrodes which make contact with the left and right palms or that potential difference data between a hand and a foot is acquired by use of an electrode which makes contact with a palm and an electrode which makes contact with the bottom of a foot. Alternatively, it is also possible that potential difference data between any body parts is acquired by use of attachable electrodes which can be attached to a body.

Then, in STEP S13, the bioelectrical impedance data (hereinafter may be abbreviated as "BI data") of the subject is acquired. That is, the BI data of the subject is calculated based on the Ohm's law by use of the current value data supplied to the body of the subject from the constant current generating circuit 16 and the potential difference data acquired in the above STEP S12 and stored in the temporary storage memory. It is also possible that a plurality of reference resistances having known and different resistance values are attached to the body in series or in parallel, potential difference data occurring due to each resistance together with potential difference data occurring in the body is acquired, and the BI data of the subject is calculated based on the ratio between each potential difference data acquired and the resistance value of the reference resistance. In this case, the BI data can still be acquired even if the current value data supplied to the body is unknown.

Then, in STEP S14, it is checked again whether the maternity mode flag is set. If it is set, the control section 15 proceeds to STEPS S141 and S142 to be described later and skips STEP S15 and proceeds to STEP S16, while if it has been cleared, the control section 15 skips STEPS S141 and S142 and proceeds to STEP S15 and then to STEP S16.

In STEP S15, the basal metabolic rate of a non-pregnant person who is the subject is acquired. As in the case of the horizontal axis in Fig. 11, this basal metabolic rate is calculated in accordance with the calculation formula disclosed in the foregoing Patent Literatures 1 and 2, i.e., BMR = A x $FFM^2$ + B x FFM + C x (1/AGE) + D x Wt + E (wherein BMR represents a basal metabolic rate, FFM represents fat free mass data, AGE represents age data, Wt represents body weight data, and A, B, C, D and E represent constants). Further, as is already well known by the foregoing Patent Literature 3 or 4, the fat free mass data of a subject is calculated based on the body weight data and body fat mass (or body fat percentage) data of the subject, and the body fat mass (or body fat percentage) data is calculated based on at least the body height data, body weight data and BI data of the subject. That is, calculation formulas for calculating these basal metabolic rate, fat free mass data and body fat mass (or body fat percentage) data are stored in the memory 18 in advance, and the basal metabolic rate of the non-pregnant subject is calculated by substituting required data out of various data stored in the temporary storage memory into the calculation formulas. Further, calculation formulas for calculating other body composition data of the subject such as a body water content, muscle amount and bone mass are also stored in the memory 18, and these body composition data are also calculated at the same time.

Then, in STEP S16, the body composition data calculated in the above STEP S15 are displayed on the display section 8 and printed in the print section 9. When the user presses the power key 10 again or a predetermined time is elapsed after completion of the above printing, the device 1 is turned off, whereby all control processings are ended.

Next, control processing when the maternity mode flag is set in the above STEP S10 will be described. In this case, the control section 15 proceeds from STEP S10 to STEP S101.

In STEP S101, the data of time elapsed after pregnancy (i.e., gestational period data) of the pregnant subject is acquired. On the display section 8, a sign or message which urges the user to enter the expected date of confinement of the subject is displayed, and the control section 15 accepts input from the numeric keypad 14. When the user enters the data of the expected date of confinement of the subject by use of the numeric keypad 14, the gestational period data of the subject is calculated based on the birth date data and the current date and time data and stored in the temporary storage memory. In this device 1, the number of weeks of pregnancy is calculated as the gestational period data. As a matter of course, the gestational period data may be the number of days of pregnancy or the number of months of pregnancy. It is also possible to directly enter the gestational period data in place of entering the data of expected date of confinement.

Then, in STEP S102, the weight data of the fetal part (fetal part weight data) of the pregnant subject is acquired. The term "fetal part weight" as used herein refers to the total of the body weight of fetus, the weight of amniotic fluid and the weight of placenta. As shown in Fig. 5 or the foregoing Patent Literature 5, the fetal part weight can be basically uniquely estimated based on the number of weeks of pregnancy. That is, in the memory 18 of the device 1, fetal part weight data corresponding to the number of weeks of pregnancy are stored, and in this STEP S102, fetal part weight data corresponding to the number of weeks of pregnancy calculated in the above STEP S101 is retrieved and stored in the temporary storage memory. The fetal part weight data can also be calculated by use of calculation formulas (function formulas represented by curves of Fig. 5) for calculating fetal part weight data from the number of weeks of pregnancy.

As described above, the gestational period data is acquired in STEP S101, and the fetal part weight data is acquired based on the acquired gestational period data in STEP S102. The fetal part weight data can also be provided by the user by directly entering data measured by a medical specialist using ultrasonotomography by use of the numeric keypad 14.

The processes of STEPS S11 to S14 subsequent to STEP S102 are as described above. However, since the maternity mode flag is set, the control section 15 proceeds to STEP S141 after STEP S14.

In STEP S141, the fat free mass data of the body of the pregnant subject is acquired. As is already well known by the foregoing Patent Literature 3 or 4, the fat free mass data of a subject is calculated based on the body weight data and body fat mass (or body fat percentage) data of the subject, and the body fat mass (or body fat percentage) data is calculated based on at least the body height data, body weight data and BI data of the subject. Thus, the fat free mass data of the body of the pregnant subject can be calculated by using the body weight data of the body as body weight data which is a parameter for calculating the fat free mass data. The body weight data of mother is calculated by subtracting the fetal part weight data from the body weight data of the pregnant subject. That is, in this STEP S141, based on the body height data, body weight data, BI data and fetal part weight data stored in the temporary storage memory, more specifically, based on the body height data, the weight data (body weight data of mother) obtained by subtracting the fetal part weight data from the body weight data and the BI data, the fat free mass data of the body of the pregnant subject is calculated and stored in the temporary storage memory.

Then, in STEP S142, the basal metabolic rate of the pregnant subject is acquired. The basal metabolic rate of the pregnant woman is calculated in accordance with a calculation formula improved from the basal metabolic rate calculation formula for non-pregnant women which is disclosed in the foregoing Patent Literatures 1 and 2 and was used in the above STEP S15, i.e., pregnant woman BMR = $A_1$ x (mother's body FFM + fetus Wt)$^2$ + $B_1$ x (mother's body FFM + fetus Wt) + $C_1$ x (1/AGE) + $D_1$ x mother's body Wt + $E_1$ (wherein pregnant woman BMR represents the basal metabolic rate of a pregnant woman, mother's body FFM represents the fat free mass data of mother, fetus Wt represents fetal part weight data, AGE represents age data, mother's body Wt represents the body weight data of mother, and $A_1$, $B_1$,

$C_1$, $D_1$ and $E_1$ represent constants). This calculation formula is a simple formula obtained by merely replacing the fat free mass data in the calculation formula for non-pregnant women with weight data (mother's body FFM + fetus Wt) obtained by adding the fetal part weight data to the fat free mass data of mother and replacing the body weight data in the calculation formula for non-pregnant women with the body weight data of mother and merely provided with appropriate constants specified by regression analysis based on a basal metabolic rate measured by use of a scentometer. That is, this calculation formula is stored in the memory 18 in advance, and in this step S142, the basal metabolic rate of the pregnant subject is calculated by substituting the fat free mass data of mother, the fetal part weight data, the age data and the body weight data of mother which are stored in the temporary storage memory into this calculation formula.

Fig. 6 shows the result of examining the accuracy of the above calculation formula for pregnant women with the help of the same pregnant women as those in the case of Figs. 10 and 11. In Fig. 6, the horizontal axis represents basal metabolic rates calculated by use of this calculation formula, the vertical axis represents basal metabolic rates measured by use of a scentometer, and the calculated basal metabolic rates and measured basal metabolic rates of the pregnant subjects are plotted as examination data. As in the case of Figs. 10 and 11, broken lines indicate matching between values on the horizontal axis and values on the vertical axis. The average line (solid line) obtained from the examination data of the subjects nearly matches the broken lines. That is, it is understood that the basal metabolic rate of a pregnant woman can be calculated accurately by use of this calculation formula.

In this STEP S142, in place of the above calculation formula, any of the following calculation formulas obtained by replacing fat free mass data in other basal metabolic rate calculation formulas disclosed in the foregoing Patent Literatures 1 and 2 with weight data (mother's body FFM + fetus Wt) obtained by adding the fetal part weight data to the fat free mass data of mother may be used.

$$\text{pregnant woman BMR} = A_2 \times (\text{mother's body FFM} + \text{fetus Wt}) + B_2 \times (1/\text{AGE})$$

$$+ \ C_2$$

$$\text{pregnant woman BMR} = A_3 \times (\text{mother's body FFM} + \text{fetus Wt})^2 + B_3 \times$$
$$(\text{mother's body FFM} + \text{fetus Wt}) + C_3 \times (1/\text{AGE}) + D_3$$

$$\text{pregnant woman BMR} = A_4 \times (\text{mother's body FFM} + \text{fetus Wt}) + B_4 \times (1/\text{AGE})$$
$$+ \ C_4 \times \text{mother's body Wt} + D_4$$

$$\text{pregnant woman BMR} = A_5 \times (\text{mother's body FFM} + \text{fetus Wt}) + B_5 \times (1/\text{AGE})$$
$$+ \ C_5 \times \text{Wt} + D_5$$

$$\text{pregnant woman BMR} = A_6 \times (\text{mother's body FFM} + \text{fetus Wt})^2 + B_6 \times$$
$$(\text{mother's body FFM} + \text{fetus Wt}) + C_6 \times (1/\text{AGE}) + D_6 \times \text{Wt} + E_6$$

In the above formulas, pregnant woman BMR represents the basal metabolic rate of a pregnant woman, mother's body FFM represents the fat free mass data of mother, fetus Wt represents fetal part weight data, AGE represents age data, mother's body Wt represents the body weight data of mother, Wt represents body weight data, and $A_2$, $B_2$, $C_2$, $A_3$, $B_3$, $C_3$, $D_3$, $A_4$, $B_4$, $C_4$, $D_4$, $E_4$, $A_5$, $B_5$, $C_5$, $D_5$, $A_6$, $B_6$, $C_6$, $D_6$ and $E_6$ represent constants.

As described above, in STEP S142, the basal metabolic rate of the subject is calculated based on the fat free mass data of mother acquired in the above STEP S141 and the age data acquired in the above STEP S7. In addition to this, as in the case of the above STEP S15, other body composition data of the subject such as a body water content, muscle amount and bone mass are also calculated at the same time. Then, the control section 15 proceeds to the above STEP S16 to display and print the calculated body composition data as described above.

Fig. 7 is a diagram showing an example of data printed out in STEP S16. Various data acquired in accordance with the flowchart of Fig. 4 are listed on the printout. "Total energy consumption" and "energy requirement" shown below "basal metabolic rate" are calculated based on a basal metabolic rate and are well known by the foregoing Nonpatent Literature 1 and other literatures. That is, the total energy consumption is calculated by multiplying the basal metabolic rate calculated in the above STEP S15 or S142 by the daily activity intensity data acquired in the above STEP S9. Further, while the energy requirement is the same as the total energy consumption for non-pregnant subjects, it is calculated by adding 350 kcal to the total energy consumption for pregnant subjects. These calculations may be performed together with calculations of other body composition data in the above STEPS S15 and S142.

As described above, in the basal metabolic rate measuring device 1 which is the first example of the present invention, age data acquiring means and body height data acquiring means are primarily constituted by the operation section 7 and the control section 15, body weight data acquiring means is primarily constituted by the body weight measuring sensor 2A and the control section 15, impedance data acquiring means is primarily constituted by the electrodes 5A, 5B, 6A and 6B, the constant current generating circuit 16, the voltage measuring circuit 17 and the control section 15, and fat free mass data acquiring means, basal metabolic rate calculating means and fetal part weight data acquiring means are primarily constituted by the control section 15 and the memory 18. The control section 15 as the fat free mass data acquiring means calculates the fat free mass data of the body of a pregnant subject based on at least body height data, body weight data, impedance data and fetal part weight data, as in STEP S141. The control section 15 as the basal metabolic rate calculating means calculates the basal metabolic rate of a pregnant subject based on at least the fat free mass data and age data of mother, as in STEP S142.

In particular, in the basal metabolic rate measuring device 1 which is the first example of the present invention, the control section 15 as the basal metabolic rate calculating means calculates the basal metabolic rate of a pregnant subject based on weight data obtained by adding fetal part weight data to the fat free mass data of mother and age data, as in STEP S142.

Further, in the basal metabolic rate measuring device 1 which is the first example of the present invention, the fetal part weight data acquiring means comprises gestational period data acquiring means and fetal part weight data determining means. The gestational period data acquiring means is primarily constituted by the operation section 7, the control section 15 and the clock circuit 19 and acquires the data of time elapsed after pregnancy of a pregnant subject, as in STEP S101. The fetal part weight data determining means is primarily constituted by the control section 15 and the memory 18 and determines the weight data of fetal part based on gestational period data, as in STEP S102.

Example 2

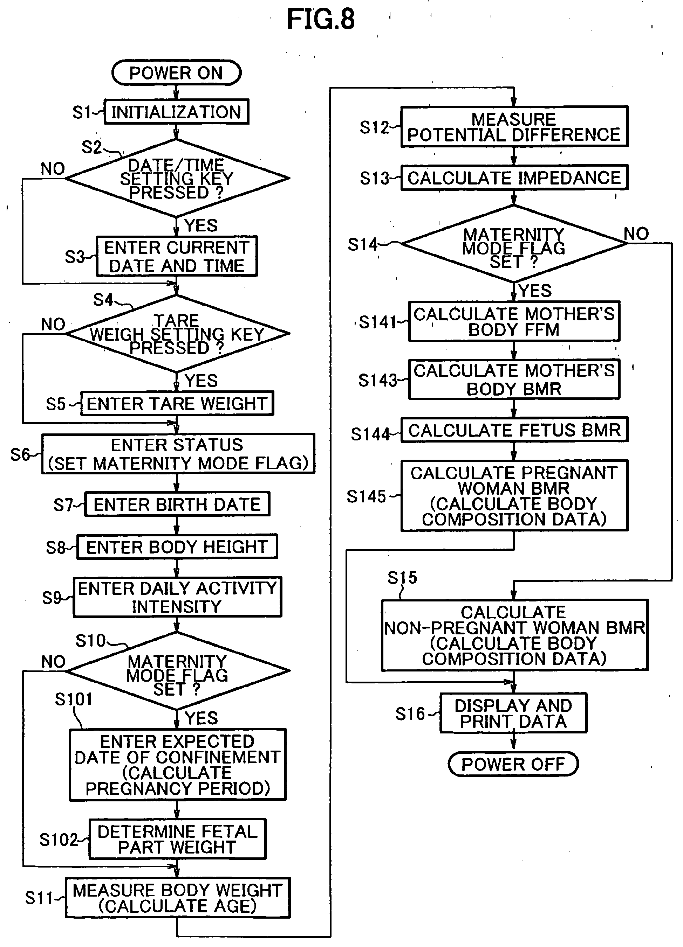

Next, a second example of the present invention will be described with reference to Figs. 8 and 9. Since the physical constitution of the basal metabolic rate measuring device of the second example is the same as that of the device 1 of the first example, Figs. 1 to 3 will be referred to as required, and detailed descriptions thereof will be omitted. Fig. 8 is a flowchart of a control processing program to be executed in the basal metabolic rate measuring device 1 as the second example. Fig. 9 is a diagram illustrating the relationship between the number of weeks of pregnancy and the basal metabolic rate of fetal part that are used in the basal metabolic rate measuring device 1 as the second example.

In the device 1 as the second example, the control processing program represented by the flowchart of Fig. 8 is executed. This control processing program is the same as the control processing program of Fig. 4 except that the processings of STEPS S143 to S145 are carried out immediately after STEP S141. Thus, descriptions of processings other than the processings of these STEPS S143 to S145 will be omitted.

After the control processing represented by STEP S141 in the flowchart of Fig. 8 is completed, i.e., the fat free mass of the body of a pregnant subject is acquired, the control section 15 proceeds to STEP S143.

In STEP S143, the basal metabolic rate of the body of the pregnant subject is acquired. The basal metabolic rate of mother is calculated in accordance with a calculation formula improved from the basal metabolic rate calculation formula for non-pregnant women which is disclosed in the foregoing Patent Literatures 1 and 2 and was used in the above STEP S15, i.e., mother's body BMR = $A_1'$ x mother's body $FFM^2$ + $B_1'$ x mother's body FFM + $C_1'$ x (1/AGE) + $D_1'$ x mother's body Wt + $E_1'$ (wherein mother's body BMR represents the basal metabolic rate of the body of a pregnant woman, mother's body FFM represents the fat free mass data of mother, AGE represents age data, mother's body Wt represents the body weight data of mother, and $A_1'$, $B_1'$, $C_1'$, $D_1'$ and $E_1'$ represent constants). This calculation formula is a simple formula obtained by merely replacing the fat free mass data in the calculation formula for non-pregnant women with the fat free mass data of mother and replacing the body weight data in the calculation formula for non-pregnant women with the body weight data of mother and merely provided with appropriate constants. That is, this calculation formula is stored in the memory 18 in advance, and in this step S143, the basal metabolic rate of the body of the pregnant subject is calculated by substituting the fat free mass data of mother, the age data and the body weight data of mother which are stored in the temporary storage memory into this calculation formula and stored in the temporary storage memory.

In this STEP S143, in place of the above calculation formula, any of the following calculation formulas obtained by replacing fat free mass data in other basal metabolic rate calculation formulas disclosed in the foregoing Patent Literatures 1 and 2 with the fat free mass data of mother may be used.

$$\text{mother's body BMR} = A_2' \times \text{mother's body FFM} + B_2' \times (1/\text{AGE}) + C_2'$$

$$\text{mother's body BMR} = A_3' \times \text{mother's body FFM}^2 + B_3' \times \text{mother's body FFM} + C_3' \times (1/\text{AGE}) + D_3'$$

$$\text{mother's body BMR} = A_4' \times \text{mother's body FFM} + B_4' \times (1/\text{AGE}) + C_4' \times \text{mother's body Wt} + D_4'$$

$$\text{mother's body BMR} = A_5' \times \text{mother's body FFM} + B_5' \times (1/\text{AGE}) + C_5' \times \text{Wt} + D_5'$$

$$\text{mother's body BMR} = A_6' \times \text{mother's body FFM}^2 + B_6' \times \text{mother's body FFM} + C_6' \times (1/\text{AGE}) + D_6' \times \text{Wt} + E_6'$$

In the above formulas, mother's body BMR represents the basal metabolic rate of the body of a pregnant woman, mother's body FFM represents the fat free mass data of mother, AGE represents age data, mother's body Wt represents the body weight data of mother, Wt represents body weight data, and $A_2'$, $B_2'$, $C_2'$, $A_3'$, $B_3'$, $C_3'$, $D_3'$, $A_4'$, $B_4'$, $C_4'$, $D_4'$, $E_4'$, $A_5'$, $B_5'$, $C_5'$, $D_5'$, $A_6'$, $B_6'$, $C_6'$, $D_6'$ and $E_6'$ represent constants.

Then, in STEP S144, the basal metabolic rate of the fetal part of the pregnant subject is acquired. The basal metabolic rate of the fetal part refers to a basal metabolic rate which increases due to the presence of a fetus in the uterus. According to Table 4 titled "Amount of increase of oxygen consumption in organs in each week of gestational period" disclosed on page 43 of the foregoing Nonpatent Literature 2, amounts of increases (ml/min) in oxygen consumption of the uterus, placenta and fetus constituting a fetal part are 0.5, 0 and 0 in the 10th week of pregnancy, 1.2, 0.5 and 1.1 in the 20th week of pregnancy, 2.2, 2.2 and 5.5 in the 30th week of pregnancy, and 3.6, 3.7 and 12.4 in the 40th week of pregnancy, respectively. When an energy amount calculated by substituting these data into a calculation formula in Table 4: 4.924 kcal x Amount of Increase in Oxygen Consumption x 1,440 minutes is taken as the basal metabolic rate of the fetal part and the relationship between the basal metabolic rate and the number of weeks of pregnancy is graphed, it is understood that between the number of weeks of pregnancy (horizontal axis) and the basal metabolic rate of the fetal part (vertical axis), there is a relationship represented by a calculation formula: fetus BMR = a x (Number of Weeks of Pregnancy) $^2$ + b x (Number of Weeks of Pregnancy) + c (wherein fetus BMR represents the basal metabolic rate of the fetal part, and a, b and c represent constants). That is, this calculation formula is stored in the memory 18 of the device 1 in advance, and in this STEP S144, the basal metabolic rate of the fetal part of the pregnant subject is calculated by substituting the number of weeks of pregnancy stored in the temporary storage memory into this calculation formula and stored in the temporary storage memory.

Then, in STEP S145, the basal metabolic rate of the pregnant subject is acquired. The basal metabolic rate of the pregnant woman is equivalent to the total of the basal metabolic rate of mother and the basal metabolic rate of the fetal part. That is, in this STEP S145, the basal metabolic rate of the pregnant subject is calculated by adding the basal metabolic rate of mother acquired in the above STEP S143 to the basal metabolic rate of the fetal part acquired in the above STEP S144 (i.e., mother's body BMR + fetus BMR). Further, in this STEP S145, in addition to calculation of the basal metabolic rate, other body composition data of the subject such as a body water content, a muscle amount and bone mass are also calculated, as in the case of the above STEP S15.

As described above, in the basal metabolic rate measuring device 1 which is the second example of the present invention, as in the case of the first example, age data acquiring means and body height data acquiring means are primarily constituted by the operation section 7 and the control section 15, body weight data acquiring means is primarily constituted by the body weight measuring sensor 2A and the control section 15, impedance data acquiring means is primarily constituted by the electrodes 5A, 5B, 6A and 6B, the constant current generating circuit 16, the voltage measuring circuit 17 and the control section 15, and fat free mass data acquiring means, basal metabolic rate calculating means and fetal part weight data acquiring means are primarily constituted by the control section 15 and the memory 18. The control section 15 as the fat free mass data acquiring means calculates the fat free mass data of the body of a pregnant subject based on at least body height data, body weight data, impedance data and fetal part weight data, as in STEP S141. The control section 15 as the basal metabolic rate calculating means calculates the basal metabolic rate of a pregnant subject based on at least the fat free mass data and age data of mother, as in STEPS S143 to S145.

In particular, in the basal metabolic rate measuring device 1 which is the second example of the present invention, the gestational period data acquiring means for acquiring the data of time elapsed after pregnancy of the pregnant subject as in STEP S101 is primarily constituted by the operation section 7, the control section 15 and the clock circuit 19, and the control section 15 as the basal metabolic rate calculating means calculates the basal metabolic rate of mother based on the fat free mass data and age data of mother as in STEP S143, calculates the basal metabolic rate of the fetal part based on the gestational period data as in STEP S144 and calculates the basal metabolic rate of the pregnant subject by totaling the basal metabolic rate of mother and the basal metabolic rate of the fetal part as in STEP S145.

Further, in the basal metabolic rate measuring device 1 which is the second example of the present invention, the fetal part weight data acquiring means comprises fetal part weight data determining means. The fetal part weight data determining means is primarily constituted by the control section 15 and the memory 18 and determines the weight data of the fetal part based on the gestational period data, as in STEP S102.

**Claims**

1. A basal metabolic rate measuring device (1) for measuring the basal metabolic rate of a pregnant subject comprising:

   age data acquiring means (14),
   body height data acquiring means (14),
   body weight data acquiring means (2A),
   impedance data acquiring means (5A, 5B, 6A, 6B),
   fat free mass data acquiring means (15), and
   basal metabolic rate calculating means (15),
   and further comprising:

   fetal part weight data acquiring means (15, 18),
   wherein
   the age data acquiring means (14) is adapted to acquire the age data of a subject,
   the body height data acquiring means (14) is adapted to acquire the body height data of the subject,
   the body weight data acquiring means (2A) is adapted to acquire the body weight data of the subject,
   the impedance data acquiring means (5A, 5B, 6A, 6B) is adapted to acquire the bioelectrical impedance data of the subject,
   the fetal part weight data acquiring means (15, 18) is adapted to acquire the weight data of the fetal part of the pregnant subject,
   the fat free mass data acquiring means (15) is adapted to calculate the fat free mass data of the body of the pregnant subject based on at least the acquired body height data, body weight data, impedance data and fetal part weight data, and the basal metabolic rate calculating means calculates the basal metabolic rate of the pregnant subject based on the age data and weight data obtained by adding the fetal part weight data to the fat free mass data of the body of the pregnant subject.

2. The basal metabolic rate measuring device of claim 1, wherein the fetal part weight data acquiring means (15, 18) comprising:

gestational period data acquiring means (14) for acquiring the data of time elapsed after pregnancy of the pregnant subject, and
fetal part weight data determining means (15, 18) for determining the weight data of the fetal part based on the gestational period data.

3. A basal metabolic rate measuring device (1) for measuring the basal metabolic rate of a pregnant subject comprising:

   age data acquiring means (14),
   body height data acquiring means (14),
   body weight data acquiring means (2A),
   impedance data acquiring means (5A, 5B, 6A, 6B),
   fat free mass data acquiring means (15) and
   basal metabolic rate calculating means (15),
   and further comprising:

      fetal part weight data acquiring means (15, 18) and
      gestational period data acquiring means (14),
      wherein
      the age data acquiring means (14) is adapted to acquire the age data of a subject,
      the body height data acquiring means (14) is adapted to acquire the body height data of the subject,
      the body weight data acquiring means (2A) is adapted to acquire the body weight data of the subject,
      the impedance data acquiring means (5A, 5B, 6A, 6B) is adapted to acquire the bioelectrical impedance data of the subject,
      the fetal part weight data acquiring means (15, 18) is adapted to acquire the weight data of the fetal part of the pregnant subject ,
      the fat free mass data acquiring means (15) is adapted to acquire the fat free mass data of the body of the pregnant subject based on at least the acquired body height data, body weight data, impedance data and fetal part weight data,
      the gestational period data acquiring means (14) is adapted to acquire the data of time elapsed after pregnancy of the pregnant subject, and
      the basal metabolic rate calculating means (15) is adapted to calculate the basal metabolic rate of the body of the pregnant subject based on the fat free mass data and age data of mother, is further adapted to calculate the basal metabolic rate of the fetal part based on the gestational period data and is further adapted of calculate the basal metabolic rate of the pregnant subject by totaling the basal metabolic rate of the body of the pregnant subject and the basal metabolic rate of the fetal part.

4. The basal metabolic rate measuring device of claim 3, wherein the fetal part weight data acquiring means (15, 18) comprises fetal part weight data determining means for determining the weight data of the fetal part based on the gestational period data acquired by the gestational period data acquiring means.

5. The basal metabolic rate measuring device of claims 2 to 4, wherein the gestational period data acquiring means comprising:

   measurement date data acquiring means for acquiring measurement date data, expected date of confinement data acquiring means for acquiring the data of the expected date of confinement of the pregnant subject, and gestational period data determining means for determining the data of time elapsed after pregnancy based on these measurement date data and data of the expected date of confinement.

**Patentansprüche**

1. Grundumsatz-Messvorrichtung (1) zum Messen des Grundumsatzes einer schwangeren Versuchsperson, umfassend:

   Altersdaten-Beschaffungsmittel (14),
   Körpergrößedaten-Beschaffungsmittel (14),
   Körpergewichtsdaten-Beschaffungsmittel (2A),
   Impedanzdaten-Beschaffungsmittel (5A, 5B, 6A, 6B),

fettfreie Massedaten-Beschaffungsmittel (15), und
Grundumsatz-Berechnungsmittel (15),
und ferner umfassend:

Fötusanteilgewichtsdaten-Beschaffungsmittel (15, 18),
wobei
das Altersdaten-Beschaffungsmittel (14) dazu geeignet ist, die Altersdaten einer Versuchsperson zu beschaffen,
das Körpergrößedaten-Beschaffungsmittel (14) dazu geeignet ist, die Körpergrößedaten der Versuchsperson zu beschaffen,
das Körpergewichtsdaten-Beschaffungsmittel (2A) dazu geeignet ist, die Körpergewichtsdaten der Versuchsperson zu beschaffen,
das Impedanzdaten-Beschaffungsmittel (5A, 5B, 6A, 6B) dazu geeignet ist, die bioelektrischen Impedanzdaten der Versuchsperson zu beschaffen,
das Fötusanteilgewichtsdaten-Beschaffungsmittel (15, 18) dazu geeignet ist, die Gewichtsdaten des Fötusanteils der schwangeren Versuchsperson zu beschaffen,
das fettfreie Massedaten-Beschaffungsmittel (15) dazu geeignet ist, die fettfreien Massedaten des Körpers der schwangeren Versuchsperson basierend auf den zumindest beschafften Körpergrößedaten, Körpergewichtsdaten, Impedanzdaten und Fötusanteilgewichtsdaten zu berechnen, und
das Grundumsatz-Berechnungsmittel den Grundumsatz der schwangeren Versuchsperson basierend auf den Altersdaten und den Gewichtsdaten, die mittels Addieren der Fötusanteilgewichtsdaten auf die fettfreien Massedaten des Körpers der schwangeren Versuchsperson erlangt werden, zu berechnen.

2.  Grundumsatz-Messvorrichtung nach Anspruch 1, wobei das Fötusanteilgewichtsdaten-Beschaffungsmittel (15, 18) umfasst:

Schwangerschaftsdauerdaten-Beschaffungsmittel (14) zum Beschaffen der Daten der Zeit, die seit der Schwangerschaft der schwangeren Versuchsperson vergangen ist, und
Fötusanteilgewichtsdaten-Bestimmungsmittel (15, 18) zum Bestimmen der Gewichtsdaten des Fötusanteils basierend auf den Schwangerschaftsdauerdaten.

3.  Grundumsatz-Messvorrichtung (1) zum Messen des Grundumsatzes einer schwangeren Versuchsperson, umfassend:

Altersdaten-Beschaffungsmittel (14),
Körpergrößedaten-Beschaffungsmittel (14),
Körpergewichtsdaten-Beschaffungsmittel (2A),
Impedanzdaten-Beschaffungsmittel (5A, 5B, 6A, 6B),
fettfreie Massedaten-Beschaffungsmittel (15), und
Grundumsatz-Berechnungsmittel (15),
und ferner umfassend:

Fötusanteilgewichtsdaten-Beschaffungsmittel (15, 18), und
Schwangerschaftsdauer-Beschaffungsmittel (14),
wobei
das Altersdaten-Beschaffungsmittel (14) dazu geeignet ist, die Altersdaten einer Versuchsperson zu beschaffen,
das Körpergrößedaten-Beschaffungsmittel (14) dazu geeignet ist, die Körpergrößendaten der Versuchsperson zu beschaffen,
das Körpergewichtsdaten-Beschaffungsmittel (2A) dazu geeignet ist, die Körpergewichtsdaten der Versuchsperson zu beschaffen,
das Impedanzdaten-Beschaffungsmittel (5A, 5B, 6A, 6B) dazu geeignet ist, die bioelektrischen Impedanzdaten der Versuchsperson zu beschaffen,
das Fötusanteilgewichtsdaten-Beschaffungsmittel (15, 18) dazu geeignet ist, die Gewichtsdaten des Fötusanteils der schwangeren Versuchsperson zu beschaffen, das fettfreie Massedaten-Beschaffungsmittel (15) dazu geeignet ist, die fettfreien Massedaten des Körpers der schwangeren Versuchsperson basierend auf den zumindest beschafften Körpergrößedaten, Körpergewichtsdaten, Impedanzdaten und Fötusanteilgewichtsdaten zu berechnen,

das Schwangerschaftsdauer-Beschaffungsmittel (14) dazu geeignet ist, die Daten der Zeit, die seit der Schwangerschaft der schwangeren Versuchsperson vergangen ist, zu beschaffen, und

das Grundumsatz-Berechnungsmittel (15) dazu geeignet ist, den Grundumsatz des Körpers der schwangeren Versuchsperson basierend auf den fettfreien Massedaten und den Altersdaten der Mutter zu berechnen und ferner dazu geeignet ist, den Grundumsatz des Fötusanteils basierend auf den Schwangerschaftsdauerdaten zu berechnen und ferner dazu geeignet ist, den Grundumsatz der schwangeren Versuchsperson durch ein Summieren des Grundumsatzes des Körpers der schwangeren Versuchsperson und des Grundumsatzes des Fötusanteils zu berechnen.

4. Grundumsatz-Messvorrichtung nach Anspruch 3, wobei das Fötusanteilgewichtsdaten-Beschaffungsmittel (15, 18) ein Fötusanteilgewichtsdaten-Bestimmungsmittel zum Bestimmen der Gewichtsdaten des Fötusanteils basierend auf den Schwangerschaftsdauerdaten, die mittels des Schwangerschaftsdauerdaten-Beschaffungsmittel beschafft wird, umfasst.

5. Grundumsatz-Messvorrichtung nach den Ansprüchen 2 bis 4, wobei das Schwangerschaftsdauerdaten-Beschaffungsmittel umfasst:

Messdatumdaten-Beschaffungsmittel zum Beschaffen von Messdatumdaten,
Beschaffungsmittel der Daten des erwarteten Datums der Entbindung zum Beschaffen der Daten des erwarteten Datums der Entbindung der schwangeren Versuchsperson, und
Schwangerschaftsdauerdaten-Bestimmungsmittel zum Bestimmen der Daten der Zeit, die seit der Schwangerschaft vergangen ist, basierend auf diesen Messdatumdaten und Daten des erwarteten Datums der Entbindung.

## Revendications

1. Dispositif de mesure de la vitesse du métabolisme basal (1) destiné à mesurer la vitesse du métabolisme basal d'une personne enceinte, comprenant :

un moyen d'acquisition de données d'âge (14),
un moyen d'acquisition de données de taille corporelle (14),
un moyen d'acquisition de données de poids corporel (2A),
un moyen d'acquisition de données d'impédance (5A, 5B, 6A, 6B),
un moyen d'acquisition de données de masse maigre (15) et
un moyen de calcul de la vitesse du métabolisme basal (15),
et comprenant en outre :

un moyen d'acquisition de données de poids de la partie foetale (15,18), où
le moyen d'acquisition de données d'âge (14) est conçu pour acquérir les données d'âge d'une personne,
le moyen d'acquisition de données de taille corporelle (14) est conçu pour acquérir les données de taille corporelle de la personne,
le moyen d'acquisition de données de poids corporel (2A) est conçu pour acquérir les données de poids corporel de la personne,
le moyen d'acquisition de données d'impédance (5A, 5B, 6A, 68) est conçu pour acquérir les données d'impédance bioélectrique de la personne,
le moyen d'acquisition de données de poids de la partie foetale (15, 18) est conçu pour acquérir les données de poids de la partie foetale de la personne enceinte,
le moyen d'acquisition de données de masse maigre (15) est conçu pour calculer les données de masse maigre du corps de la personne enceinte d'après au moins les données de taille corporelle, les données de poids corporel, les données d'impédance et les données de poids de la partie foetale acquises, et
le moyen de calcul de la vitesse du métabolisme basal calcule la vitesse du métabolisme basal de la personne enceinte d'après les données d'âge et les données de poids obtenues par l'addition des données de poids de la partie foetale et des données de masse maigre du corps de la personne enceinte.

2. Dispositif de mesure de la vitesse du métabolisme basal selon la revendication 1, où
le moyen d'acquisition des données de poids de la partie foetale (15, 18) comprend :
un moyen d'acquisition de données de la période de gestation (14) destiné à acquérir les données de temps écoulé après la grossesse de la personne enceinte, et

un moyen de détermination des données de poids de la partie foetale (15, 18) destiné à déterminer les données de poids de la partie foetale d'après les données de la période de gestation.

3. Dispositif de mesure de la vitesse du métabolisme basal (1) destiné à mesurer la vitesse du métabolisme basal d'une personne enceinte comprenant-:-

un moyen d'acquisition de données d'âge (14),
un moyen d'acquisition de données de taille corporelle (14),
un moyen d'acquisition de données de poids corporel (2A),
un moyen d'acquisition de données d'impédance (5A, 5B, 6A, 6B),
un moyen d'acquisition de données de masse maigre (15) et
un moyen de calcul de la vitesse du métabolisme basal (15),
et comprenant en outre :

un moyen d'acquisition des données de poids de la partie foetale (15, 18) et
un moyen d'acquisition des données de la période de gestation (14), où
le moyen d'acquisition de données d'âge (14) est conçu pour acquérir les données d'âge d'une personne,
le moyen d'acquisition de données de taille corporelle (14) est conçu pour acquérir les données de taille corporelle de la personne,
le moyen d'acquisition de données de poids corporel (2A) est conçu pour acquérir les données de poids corporel de la personne,
le moyen d'acquisition de données d'impédance (5A, 5B, 6A, 6B) est conçu pour acquérir les données d'impédance bioélectrique de la personne,
le moyen d'acquisition de données de poids de la partie foetale (15, 18) est conçu pour acquérir les données de poids de la partie foetale de la personne enceinte, le moyen d'acquisition de données de masse malgre (15) est conçu pour calculer les données de masse malgre du corps de la personne enceinte d'après au moins les données de taille corporelle, les données de poids corporel, les données d'impédance et les données de poids de la partie foetale acquises,
le moyen d'acquisition de données de la période de gestation (14) est conçu pour acquérir les données de temps écoulé après la grossesse de la personne enceinte, et
le moyen de calcul de la vitesse du métabolisme basal (15) est conçu pour calculer la vitesse du métabolisme basal du corps de la personne enceinte d'après les données de masse maigre et les données d'âge de la mère, est conçu en outre pour calculer la vitesse du métabolisme basal de la partie foetale d'après les données de la période de gestation et est conçu en outre pour calculer la vitesse du métabolisme basal de la personne enceinte en faisant la somme de la vitesse du métabolisme basal du corps de la personne enceinte et la vitesse du métabolisme basal de la partie foetale.

4. Dispositif de mesure de la vitesse du métabolisme basal selon la revendication 3, où le moyen d'acquisition de données de poids de la partie foetale (15, 18) comprend le moyen de détermination des données de poids de la partie foetale destiné à déterminer les données de poids de la partie foetale d'après les données de la période de gestation acquises par le moyen d'acquisition de données de la période de gestation.

5. Dispositif de mesure de la vitesse du métabolisme basal selon les revendications 2 à 4, où le moyen d'acquisition de données de la période de gestation comprend :

un moyen d'acquisition de données de date de mesure destiné à acquérir des données de date de mesure, un moyen d'acquisition de données de date attendue d'accouchement destiné à acquérir les données de date attendue d'accouchement de la personne enceinte, et
un moyen de détermination des données de la période de gestation destiné à déterminer les données de temps écoulé après la grossesse d'après ces données de date de mesure et ces données de date attendue d'accouchement.

# FIG.1

## FIG.2

| | 5A | 5B | 6A | 6B |

**16** CONSTANT CURRENT GENERATING CIRCUIT

**17** VOLTAGE MEASURING CIRCUIT

**8** DISPLAY SECTION

**2A** BODY WEIGHT MEASURING SENSOR

**15** CONTROL SECTION

**7** OPERATION SECTION

**9** PRINT SECTION

**20** POWER SOURCE → TO EACH SECTION

**19** CLOCK CIRCUIT

**18** MEMORY

## FIG.3

**10** ON/OFF

**11** TARE WEIGHT SETTING KEY

**12** DATE/TIME SETTING KEY

**7**

MALE — STANDARD, ATHLETE

FEMALE — STANDARD, ATHLETE

MATERNITY

7 8 9
4 5 6
1 2 3
0 . CE

**13**

**14**

# FIG.4

```
        ( POWER ON )
              │
              ▼
S1 ──┤   INITIALIZATION   │
              │
              ▼
S2        ╱DATE/╲
       ╱TIME SETTING KEY╲──NO──┐
       ╲  PRESSED ?   ╱         │
          ╲      ╱               │
            │YES                 │
            ▼                    │
S3 ──┤  ENTER CURRENT   │        │
     │  DATE AND TIME   │        │
            │◄───────────────────┘
            ▼
S4      ╱  TARE  ╲
     ╱WEIGHT SETTING KEY╲──NO──┐
     ╲    PRESSED?    ╱         │
        ╲        ╱               │
            │YES                 │
            ▼                    │
S5 ──┤ ENTER TARE WEIGHT │       │
            │◄───────────────────┘
            ▼
S6 ──┤  ENTER STATUS    │
     │ (SET MATERNITY   │
     │   MODE FLAG)     │
            │
            ▼
S7 ──┤ ENTER BIRTH DATE │
     │ (CALCULATE AGE)  │
            │
            ▼
S8 ──┤ ENTER BODY HEIGHT │
            │
            ▼
S9 ──┤  ENTER DAILY     │
     │ ACTIVITY INTENSITY│
            │
            └──────────────►
```

```
      ┌────────────────────────►
      │
      ▼
S10   ╱ MATERNITY MODE ╲──NO──┐
   ╱   FLAG SET ?    ╲         │
      ╲          ╱               │
         │YES                    │
         ▼                       │
S101 ┤ ENTER EXPECTED DATE │     │
     │  OF CONFINEMENT     │     │
     │   (CALCULATE        │     │
     │ PREGNANCY PERIOD)   │     │
         │                       │
         ▼                       │
S102 ┤  DETERMINE FETAL   │      │
     │   PART WEIGHT      │      │
         │◄──────────────────────┘
         ▼
S11 ─┤  MEASURE BODY     │
     │    WEIGHT         │
         │
         ▼
S12 ─┤ MEASURE POTENTIAL │
     │   DIFFERENCE      │
         │
         ▼
S13 ─┤   CALCULATE       │
     │   IMPEDANCE       │
         │
         ▼
S14   ╱ MATERNITY MODE ╲──NO──┐
   ╱   FLAG SET ?    ╲         │
      ╲          ╱               │
         │YES                    │
         ▼                       │
S141 ┤CALCULATE MOTHER'S │       │
     │   BODY FFM        │       │
         │                       │
         ▼                       │
S142 ┤CALCULATE PREGNANT │       │
     │  WOMAN BMR        │       │
     │ (CALCULATE BODY   │       │
     │ COMPOSITION DATA) │       │
         │◄──────────────────────┘
         ▼
S15 ─┤ CALCULATE NON-    │
     │PREGNANT WOMAN BMR │
     │ (CALCULATE BODY   │
     │ COMPOSITION DATA) │
         │
         ▼
S16 ─┤   DISPLAY AND     │
     │   PRINT DATA      │
         │
         ▼
     ( POWER OFF )
```

## FIG.5

## FIG.6

# FIG.7

| | | |
|---|---|---|
| DATE AND TIME OF MEASUREMENT | 11/9/2004 | 10:27 AM |
| STATUS | | MATERNITY |
| NUMBER OF WEEKS OF PREGNANCY | | 24 WEEKS AND 5 DAYS |
| AGE | | 35 YEARS OLD |
| BODY HEIGHT | | 159 cm |
| BODY WEIGHT | | 52.4 kg |
| TARE WEIGHT | | 0.5 kg |
| BODY WEIGHT OF FETUS | | 1.201 kg |
| WEIGHT OF AMNIOTIC FLUID | | 0.534 kg |
| WEIGHT OF PLACENTA | | 0.265 kg |
| IMPEDANCE | | 580 Ω |
| BODY FAT PERCENTAGE | | 27.5 % |
| BODY FAT MASS | | 14.4 kg |
| FAT FREE MASS | | 38.0 kg |
| BODY WATER CONTENT | | 27.8 kg |
| BASAL METABOLIC RATE | | 1163 kcal/day |
| TOTAL ENERGY CONSUMPTION | | 1512 kcal/day |
| ENERGY REQUIREMENT | | 1862 kcal/day |

# FIG.8

```
                 ( POWER ON )
                      │
          S1─┤ INITIALIZATION │
                      │
          S2─╮
      NO   ╱ DATE/TIME ╲
    ◄──────╱ SETTING KEY ╲
           ╲ PRESSED ? ╱
             ╲       ╱
                │ YES
          S3─┤ ENTER CURRENT
             │ DATE AND TIME │
                │
          S4─╮
      NO   ╱    TARE    ╲
    ◄──────╱ WEIGH SETTING KEY ╲
           ╲ PRESSED ? ╱
             ╲       ╱
                │ YES
          S5─┤ ENTER TARE WEIGHT │
                │
          S6─┤ ENTER STATUS
             │ (SET MATERNITY MODE FLAG) │
                │
          S7─┤ ENTER BIRTH DATE │
                │
          S8─┤ ENTER BODY HEIGHT │
                │
          S9─┤ ENTER DAILY ACTIVITY
             │ INTENSITY │
                │
         S10─╮
      NO   ╱ MATERNITY ╲
    ◄──────╱ MODE FLAG ╲
           ╲ SET ? ╱
             ╲   ╱
                │ YES
     S101─┤ ENTER EXPECTED
          │ DATE OF CONFINEMENT
          │ (CALCULATE
          │ PREGNANCY PERIOD) │
                │
     S102─┤ DETERMINE FETAL
          │ PART WEIGHT │
                │
     S11─┤ MEASURE BODY WEIGHT
         │ (CALCULATE AGE) │
```

```
         S12─┤ MEASURE
             │ POTENTIAL DIFFERENCE │
                │
         S13─┤ CALCULATE IMPEDANCE │
                │
         S14─╮                          NO
           ╱ MATERNITY ╲
           ╱ MODE FLAG ╲────────►
           ╲ SET ? ╱
             ╲   ╱
                │ YES
     S141─┤ CALCULATE MOTHER'S
          │ BODY FFM │
                │
     S143─┤ CALCULATE MOTHER'S
          │ BODY BMR │
                │
     S144─┤ CALCULATE FETUS BMR │
                │
     S145─┤ CALCULATE PREGNANT
          │ WOMAN BMR
          │ (CALCULATE BODY
          │ COMPOSITION DATA) │
                │
         S15─┤ CALCULATE
             │ NON-PREGNANT WOMAN BMR
             │ (CALCULATE BODY
             │ COMPOSITION DATA) │
                │
         S16─┤ DISPLAY AND
             │ PRINT DATA │
                │
            ( POWER OFF )
```

23

## FIG.9

$$y = ax^2 + bx + c$$

FETAL PART BMR(kcal/day) vs WEEKS OF PREGNANCY

## FIG.10

MEASURED BMRS(kcal/day) vs CALCULATED BMRS BASED ON METHOD OF MINISTRY OF HEALTH, LABOR AND WELFARE(kcal/day)

# FIG.11

MEASURED BMRS(kcal/day)

2000

1750

1500

1250

1000

750

500

500  750  1000  1250  1500  1750  2000

CALCULATED BMRS BASED ON METHOD OF
PATENT LITERATURE 1(kcal/day)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002112982 A
- JP 2002172099 A
- JP 5049050 A
- JP 2835662 B
- JP 2003102696 A
- EP 1195138 A1
- EP 1279367 A1

**Non-patent literature cited in the description**

- 6th revised edition Nutritional Requirements of Japanese Reference Dietary Intake. Health and Nutrition Information Association. Daiichi Shuppan Kabushiki Kaisha, 10 September 1999, 35-36
- **Masaharu Onaka.** Nutrition Guidance for Pregnant Women/Nutrition Guidance for Normal Pregnant Women 2 Energy. *MEDICUS LIBRARY 5 Nutrition Guidance Recommended for Mother and Child,* 15 April 1997, 40-44